(19) Europäisches Patentamt European Patent Office Office européen des brevets

(11) **EP 4 657 372 A1**

(12) **EUROPEAN PATENT APPLICATION**

(43) Date of publication:
03.12.2025 Bulletin 2025/49

(21) Application number: 25172332.6

(22) Date of filing: 24.04.2025

(51) International Patent Classification (IPC):
**G06T 7/11** (2017.01)       **G06T 7/187** (2017.01)
**G06T 7/00** (2017.01)       **G06T 7/12** (2017.01)

(52) Cooperative Patent Classification (CPC):
**G06T 7/12; G06T 7/0004;** G06T 2207/10116;
G06T 2207/20061; G06T 2207/20081;
G06T 2207/30168

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: 28.05.2024  KR 20240069329
16.10.2024  KR 20240141042

(71) Applicant: Doosan Enerbility Co., Ltd.
Seongsan-gu
Changwon-si, Gyeongsangnam-do 51711 (KR)

(72) Inventors:
• YU, Jun Sang
13607 Seongnam-si, Gyeonggi-do (KR)
• JIN, Sung Wook
06114 Seoul (KR)
• PARK, Gung Hul
06625 Seoul (KR)

(74) Representative: BCKIP Part mbB
MK1
Landsbergerstraße 98, 3.Stock
80339 München (DE)

(54) **DEVICE FOR INSPECTING DEFECT IN WELD ON BASIS OF RADIOGRAPHIC TESTING AND METHOD THEREFOR**

(57)    Proposed are a device for inspecting a defect in a weld based on radiographic testing and a method therefor, and the method includes a step of evaluating a quality of the radiographic image according to the number of counted wires of the image quality indicator, a step of performing image processing so as to highlight features of the defect in the welded part in the reading region when the quality of the radiographic image satisfies a preset reference value, a step of detecting the defect in the welded part in the reading region, and a step of outputting a defect report that includes a defect location, a defect area, and a defect type according to the detected defect.

EP 4 657 372 A1

**Description**

BACKGROUND

Field of the Invention

The present disclosure relates to a technology for inspecting a defect in a weld and, more particularly, to a device for inspecting a defect in a weld on the basis of radiographic testing (RT) and a method therefor.

Description of the Related Art

Radiographic testing (RT) is a testing method that selects radiation such as X-rays or gamma rays in accordance with usage conditions and purpose, passes the radiation through a test specimen, and forms an image on an X-ray film, so as to detect defects inside the test specimen, and is currently the most widely used non-destructive testing method for detecting internal defects.

SUMMARY

An objective of the present disclosure is to provide a device for inspecting a defect in a weld on the basis of radiographic testing and a method therefor.

According to a preferred exemplary embodiment of the present disclosure to achieve the above-described objective, there is provided a method for inspecting a defect in a weld, the method including: performing, by a reading region processing unit, image processing so as to highlight features of a reading region including a welded part of a target object in a radiographic image; detecting, by the reading region processing unit, the reading region in the radiographic image; performing, by an image quality processing unit, image processing so as to highlight features of an image quality indicator in the radiographic image; counting, by the image quality processing unit, the number of wires of the image quality indicator in the radiographic image; evaluating, by the image quality processing unit, a quality of the radiographic image according to the number of counted wires of the image quality indicator; performing, by a defect processing unit, image processing so as to highlight features of the defect in the welded part in the reading region when the quality of the radiographic image satisfies a preset reference value; detecting, by the defect processing unit, the defect in the welded part in the reading region; and outputting, by an output unit, a defect report including a defect location, a defect area, and a defect type according to the detected defect.

In the detecting of the reading region, the reading region processing unit may extract signals representing levels of pixels of the radiographic image through a local extremum point search-based detection model, detect edges representing a weld bead in the extracted signals from maximum and minimum points of the extracted signals, and detect an area occupied by the edges representing the weld bead in the radiographic image as the reading region through a bounding box.

In the detecting of the reading region, the reading region processing unit may detect an area occupied by the reading region in the radiographic image by using a bounding box through a reading region detection model, which is a learning model.

The detecting of the reading region may include: extracting, by the reading region processing unit, signals that represent levels of pixels of the radiographic image through a local extremum point search-based detection model, detecting edges that represent a weld bead in the extracted signals from maximum and minimum points of the signals, and detecting an area occupied by the edges that represent the weld bead in the radiographic image as the reading region through a first bounding box; detecting, by the reading region processing unit, an area occupied by the reading region through a second bounding box by using a reading region detection model; and detecting, by the reading region processing unit, an intersection area of the first bounding box and the second bounding box as a final reading region.

The detecting of the number of wires of the image quality indicator may include: specifying, by the image quality processing unit, a text area, which is an area occupied by a text of the image quality indicator in the radiographic image; specifying, by the image quality processing unit, a wire area, which is an area occupied by the wires of the image quality indicator on the basis of the text area; and counting, by the image quality processing unit, the number of wires of the image quality indicator in the wire area.

The counting of the number of wires of the image quality indicator in the wire area may include: performing, by the image quality processing unit, a Hough Transform on the wire area, so as to create an image of the wire area having a plurality of lines; clustering, by the image quality processing unit, the plurality of lines on the basis of a density of the plurality of lines, so as to derive one or more line clusters; and counting, by the image quality processing unit, the number of wires of the image quality indicator according to the number of derived line clusters.

The detecting of the number of wires of the image quality indicator may include: using the image quality indicator detection model, by the image quality processing unit, so as to detect a wire area that is an area occupied by a plurality of wires of the image quality indicator in the radiographic image through a bounding box; and using a count model, by the image quality processing unit, so as to detect each count wire area that is an area occupied by each of the plurality of wires of the image quality indicator in the wire area through a bounding box, and count the number of wires of the image quality indicator according to the number of detected count wire areas.

In the detecting the defect of the welded part, the defect processing unit may derive a defect vector by performing weight calculations in which weights that have been learned are applied to the reading region of the radiographic image, and the defect vector may include a defect bounding box for indicating an area occupied by the defect in the reading region of the radiographic image and a defect class for indicating a type of the detected defect.

The method may further include: before the performing of the image processing so as to highlight the features of the reading region, loading, by a training unit, training data including a training radiographic image and a target vector; inputting, by the training unit, the training radiographic image into a defect detection model having weights that have not been learned; performing, by the defect detection model, weight calculations in which the weights that have not been learned are applied to the training radiographic image, so as to derive a defect vector including a defect bounding box for detecting an area occupied by the defect in the training radiographic image and a defect class for indicating a type of the detected defect; calculating, by the training unit, a loss representing a difference between the target vector and the defect vector through a loss function; and performing, by the training unit, optimization for modifying the weights of the defect detection model so as to maximally reduce the calculated loss.

The training radiographic image may include a radiographic image, which is of a target object having a defect in a welded part and generated by synthesizing an image of a defect part with a radiographic image of a target object having no defect in a welded part, and the target vector may include a target bounding box for indicating the area occupied by the defect in the training radiographic image and the target class for indicating the defect existing in the training radiographic image.

According to the preferred exemplary embodiment of the present disclosure to achieve the above-described objective, there is provided a device for inspecting a defect in a weld, the device including: a reading region processing unit for performing image processing so as to highlight features of a reading region that includes a welded part of a target object in a radiographic image, and detecting the reading region in the radiographic image; an image quality processing unit for performing image processing so as to highlight features of an image quality indicator in the radiographic image, count the number of wires of the image quality indicator in the radiographic image, and evaluate quality of the radiographic image according to the number of counted wires of the image quality indicator; a defect processing unit for performing image processing so as to highlight features of the defect in the welded part in the reading region when the quality of the radiographic image satisfies a preset reference value, and detecting the defect in the welded part in the reading region; and an output unit for outputting a defect report that includes a defect location, a defect area, and a defect type according to the detected defect.

The reading region processing unit may extract signals representing levels of pixels of the radiographic image through a local extremum point search-based detection model, detect edges representing a weld bead in the extracted signals from maximum and minimum points of the extracted signals, and detect an area occupied by the edges representing the weld bead in the radiographic image as the reading region through a bounding box.

The reading region processing unit may detect an area occupied by the reading region in the radiographic image by using a bounding box through a reading region detection model, which is a learning model.

The reading region processing unit may extract signals representing levels of pixels of the radiographic image through a local extremum point search-based detection model, detect edges representing a weld bead in the extracted signals from maximum and minimum points of the signals, detect an area occupied by the edges representing the weld bead in the radiographic image as the reading region through a first bounding box, detect an area occupied by the reading region through a second bounding box by using a reading region detection model, and detect an intersection area of the first bounding box and the second bounding box as a final reading region.

The image quality processing unit may specify a text area, which is an area occupied by a text of the image quality indicator in the radiographic image, specify a wire area, which is an area occupied by the wires of the image quality indicator on the basis of the text area, and count the number of wires of the image quality indicator in the wire area.

The image quality processing unit may perform a Hough Transform on the wire area, so that an image of the wire area having a plurality of lines is created, cluster the plurality of lines on the basis of a density of the plurality of lines, so that one or more line clusters are derived, and count the number of wires of the image quality indicator according to the number of derived line clusters.

The image quality processing unit may use the image quality indicator detection model so that a wire area that is an area occupied by a plurality of wires of the image quality indicator in the radiographic image is detected through a bounding box, use a count model so that each count wire area that is an area occupied by each of the plurality of wires of the image quality indicator in the wire area is detected through a bounding box, and count the number of wires of the image quality indicator according to the number of detected count wire areas.

The defect processing unit may derive a defect vector by performing weight calculations in which weights that have been learned are applied to the reading region of the radiographic image, and the defect vector may include a defect bounding box for indicating an area occupied by the defect in the reading region of the radiographic image and a defect class for indicating a type of the detected defect.

The device may further include a training unit for loading training data that includes a training radiographic image and a target vector, inputting the training radiographic image into a defect detection model having weights that have not been learned, allowing the defect detection model to perform weight calculations in which the weights that have not been learned are applied to the training radiographic image, so as to derive a defect vector that includes a defect bounding box configured to detect an area occupied by the defect in the training radiographic image and a defect class configured to indicate a type of the detected defect, and then calculating a loss representing a difference between the target vector and the defect vector through a loss function and performing optimization configured to modify the weights of the defect detection model so as to maximally reduce the calculated loss.

The training radiographic image may include a radiographic image, which is of a target object having a defect in a welded part and generated by synthesizing an image of a defect part with a radiographic image of a target object having no defect in a welded part, and the target vector may include a target bounding box for indicating the area occupied by the defect in the training radiographic image and the target class for indicating the defect existing in the training radiographic image.

Compared to a method for performing evaluation by an inspector with his or her naked eyes, the embodiment according to the present disclosure provides an effect that inspection time may be shortened and objective results may be obtained.

BRIEF DESCRIPTION OF THE DRAWINGS

FIG. 1 is a view illustrating a configuration of a system for inspecting a defect in a weld on the basis of radiographic testing according to an exemplary embodiment of the present disclosure.

FIG. 2 is a view illustrating a configuration of a device for inspecting a defect in a weld on the basis of radiographic testing according to the exemplary embodiment of the present disclosure.

FIG. 3 is a flowchart illustrating a method for generating a defect detection model through learning according to the exemplary embodiment of the present disclosure.

FIG. 4 is a flowchart illustrating a method for performing radiographic testing (RT) by deriving a digitized radiographic image according to the exemplary embodiment of the present disclosure.

FIG. 5 is a flowchart illustrating a method for inspecting a defect in a weld on the basis of radiographic testing according to the exemplary embodiment of the present disclosure.

FIG. 6, FIG. 7, FIG. 8, FIG. 9, FIG. 10, and FIG. 11 are screen examples illustrating the method for inspecting the defect in the weld on the basis of the radiographic testing according to the exemplary embodiment of the present disclosure.

FIG. 12 is a view illustrating a computing device according to the exemplary embodiment of the present disclosure.

DETAILED DESCRIPTION

The present disclosure may be modified in various ways and may have various exemplary embodiments, and thus a specific exemplary embodiment will be exemplified and described in detail in the detailed description. However, this is not intended to limit the present disclosure to a particular disclosed form. On the contrary, the present disclosure is to be understood to include all various transformations, equivalents, and substitutes that may be included within the idea and technical scope of the present disclosure.

The terminology used in the present disclosure is for the purpose of describing particular exemplary embodiments only and is not intended to be limiting. As used herein, the singular forms are intended to include the plural forms as well, unless the context clearly indicates otherwise. In addition, it will be further understood that the terms "comprise", "include", "have", etc. when used in the present disclosure, specify the presence of stated features, integers, steps, operations, elements, components, parts, and/or combinations thereof, but do not preclude the possibility of the presence or addition of one or more other features, integers, steps, operations, elements, components, parts, and/or combinations thereof.

Particularly, the terms or words used in the present specification and claims are not to be construed as being limited to their ordinary or dictionary meanings, and should be interpreted as meanings and concepts corresponding to the technical spirit of the present disclosure based on the principle that inventors may properly define the concept of each term in order to best describe their embodiments. In particular, in the exemplary embodiment of the present disclosure, "estimation" means deriving a result calculated according to what the learning model (LM: Machine learning model/Deep learning model) has learned.

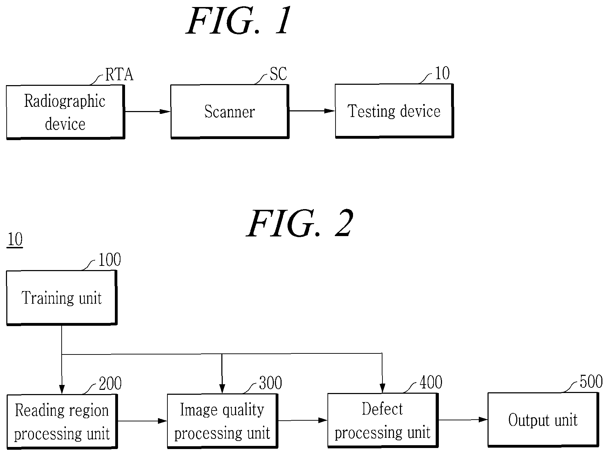

First, a device for inspecting a defect in a weld on the basis of radiographic testing according to the exemplary embodiment of the present disclosure will be described. FIG. 1 is a view illustrating a configuration of a system for inspecting the defect in the weld on the basis of the radiographic testing according to the exemplary embodiment of the present disclosure. FIG. 2 is a view illustrating a configuration of a device for inspecting the defect in the weld on the basis of the radiographic testing according to the exemplary embodiment of the present disclosure.

Referring to FIG. 1, the system for inspecting the defect in the weld on the basis of the radiographic testing according to the exemplary embodiment of the present disclosure includes a radiographic device RTA, a scanner SC, and a testing device 10.

The radiographic device RTA is for performing radiography of a target object to which an image quality indicator (IQI) is attached, so as to derive a radiographic testing film in which the target object is radiographed. Here, the target object may be exemplified as a pipe, tube, etc.

The scanner SC is for scanning a radiographic testing film, so as to generate a digitized radiographic image. As such, when the digitized radiographic image is generated, the generated radiographic image is input into the testing device 10. In this case, the radiographic image may be input directly from the scanner SC or may be stored in another storage medium and then input from the corresponding storage medium.

The testing device 10 is for testing a defect in a weld in a radiographic image. To this end, the testing device 10 includes a training unit 100, a reading region processing unit 200, an image quality processing unit 300, a defect processing unit 400, and an output unit 500.

The training unit 100 is fundamentally for training a learning model through learning (i.e., Deep Learning or Machine Learning). In addition, the training unit 100 may perform continual learning on the learning model. The learning model according to the exemplary embodiment of the present disclosure includes a reading region detection model, an image quality indicator detection model, a count model, and a defect detection model. The reading region detection model is trained to detect a reading region, which is a region including a weld bead in a radiographic image. The image quality indicator detection model is trained to detect a wire area WA including a plurality of wires of an image quality indicator in the radiographic image. The count model is trained to detect each individual wire in the wire area WA. The defect detection model is trained to detect a defect in a welded part in the reading region.

According to the exemplary embodiment of the present disclosure, examples of the learning model may include convolutional neural network (CNN), You Only Look Once (YOLO), region-based convolutional neural network (RCNN), Faster RCNN, etc. The learning model, including the reading region detection model, image quality indicator detection model, count model, and defect detection model, includes a plurality of layers (or modules) connected to each other, and the plurality of layers (or modules) is configured for a plurality of calculations. In addition, the plurality of layers (or modules) is connected to each other through weights W. That is, an output according to a calculation result of any one layer (or module) is applied with a weight and used as an input for a calculation of the next layer thereof. The detection model DM derives an output by performing the plurality of calculations applied with the weights between the plurality of layers (or modules) on input data. In other words, the learning model performs the plurality of calculations linked by the weights between the plurality of layers (or modules). The plurality of calculations linked by the weights between the plurality of layers (or modules) of such a learning model is referred to as "weight calculations."

In particular, the training unit 100 performs continual learning on the learning model including the above-described reading region detection model, image quality indicator detection model, count model, and defect detection model. Such continual learning is to continually update a corresponding learning model by using new training data. The training unit 100 may evaluate the performance of each of the corresponding learning models by using new training data. For example, the training unit 100 may measure the degree of performance degradation by using an average forgetting index. Consequently, when a result of performance evaluation of each of the learning models exceeds a threshold value of the performance degradation, the training unit 100 may update the corresponding learning model by using new training data.

The reading region processing unit 200 is for detecting the reading region ROI in the radiographic image after performing image processing so as to highlight features of the reading region ROI including the welded part of the target object in the radiographic image.

The image quality processing unit 300 is for performing image processing to highlight the features of an image quality indicator in the radiographic image, counting the number of wires of the image quality indicator in the radiographic image, and then evaluating the quality of the radiographic image on the basis of the number of counted wires of the image quality indicator.

The defect processing unit 400 is for performing image processing to highlight the features of the defect in the welded part in the reading region ROI, and then detecting the defect in the welded part in the reading region ROI.

The output unit 500 is for outputting a defect report including a location of the defect, an area of the defect, and a type of the defect when the defect in the welded part is detected.

The specific operations of the testing device 10 including the above-described reading region processing unit 200, image quality processing unit 300, defect processing unit 400, and output unit 500 will be described in more detail below.

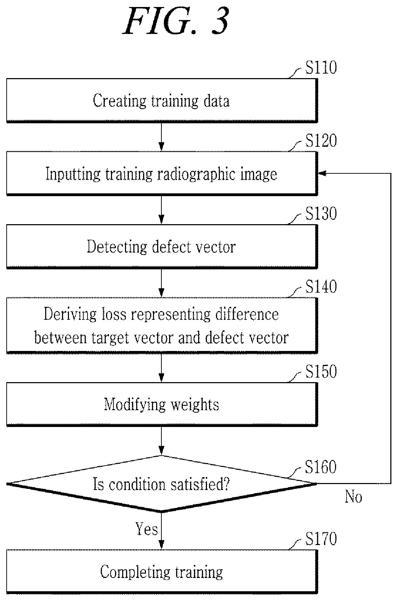

Next, a way of describing a method for generating a defect detection model through learning according to the exemplary embodiment of the present disclosure will be provided. FIG. 3 is a flowchart illustrating the method for generating the defect detection model through the learning according to the exemplary embodiment of the present disclosure.

Referring to FIG. 3, in step S110, a training unit 100 loads training data prepared in advance. The training data includes a training radiographic image RI and a target vector. The training radiographic image RI is a radiographic image of a target object having a welded part. In particular, the training radiographic image RI may be a radiographic image of a target object having a defect in a welded part, or may be a radiographic image of a target object having no defect in a welded part. In particular, when the amount of training data of a class representing a specific type of defect is insufficient, a radiographic image of a target object having a defect in a welded part may be generated by synthesizing an image of a defect part with a radiographic image of a target object having no defect in a welded part.

The target vector includes: a target bounding box (e.g., GT) representing an area occupied by a defect in a training radiographic image; and a target class representing a class of the defect present in the training radiographic image. Here, the target bounding box (e.g., GT) may be expressed as a bounding box having center coordinates, a width, and a height.

Next, in step S120, the training unit 100 inputs the training radiographic image in a defect detection model having weights that have not been learned. Then, in step S130, the defect detection model derives a defect vector by performing weight calculations in which the weights that have not been learned are applied to the training radiographic image. The defect vector includes a defect bounding box (e.g., BB) and a defect class. The defect bounding box detects an area occupied by a defect in the training radiographic image. The defect bounding box (e.g., BB) may be expressed by center coordinates, a width, and a height of this bounding box. The defect class represents a class of the detected defect. The class of defect is for classifying the type of defect, and for example, the class of defect may be exemplified as pores, slag mixing, incomplete penetration, undercut, overlap, and welding cracks.

Subsequently, in step S140, the training unit 100 calculates a loss indicating a difference between the target vector and the defect vector through a loss function. Then, in step S150, the training unit 100 performs optimization to modify the weights of the defect detection model so as to maximally reduce the loss derived through the loss function.

Next, in step S160, the training unit 100 determines whether a training completion condition is satisfied. According to the exemplary embodiment, the training completion condition may be a case when a predetermined learning rate is exceeded and the loss calculated previously in step S140 converges to be less than or equal to a preset target value. As a result of determination in step S160, when the training completion condition is not satisfied, steps S120 to S150 described above are iteratively performed by using a plurality of training data different from each other. In contrast, as a result of the determination in step S160, when the training completion condition is satisfied, the training unit 100 completes the training of the defect detection model in step S 170.

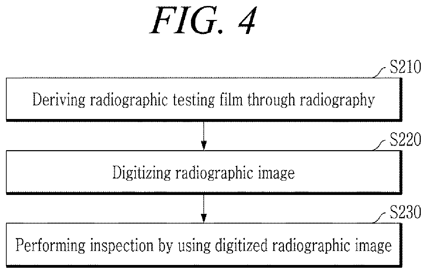

Next, a method for performing radiographic testing (RT) by deriving a digitized radiographic image according to the exemplary embodiment of the present disclosure will be described. Next, FIG. 4 is a flowchart illustrating the method for performing the radiographic testing (RT) by deriving the digitized radiographic image according to the exemplary embodiment of the present disclosure.

Referring to FIG. 4, in step S210, a radiographic testing device RTA derives a radiographic testing film by performing radiography while having an image quality indicator (IQI) attached to a target object on which welding is performed. Here, the target object may be exemplified as a pipe, tube, etc.

Then, in step S220, a scanner SC scans the radiographic testing film, so as to generate a digitized radiographic image.

After the radiographic image is generated, the testing device 10 receives input of the digitized radiographic image in step S230, and performs inspection of a welded part by analyzing the input radiographic image. In this case, the digitized radiographic image may be input directly from the scanner SC or input through other media. In addition, information about the target object of the radiographic image may be input by a user.

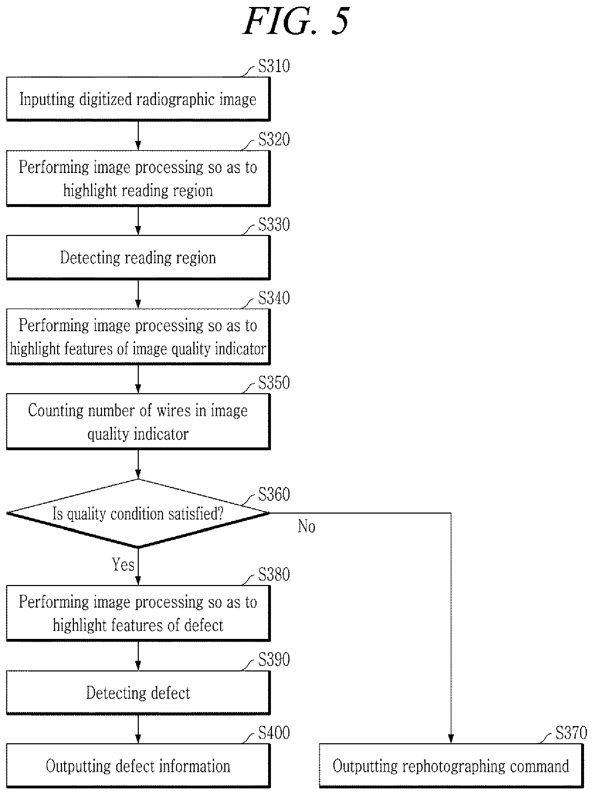

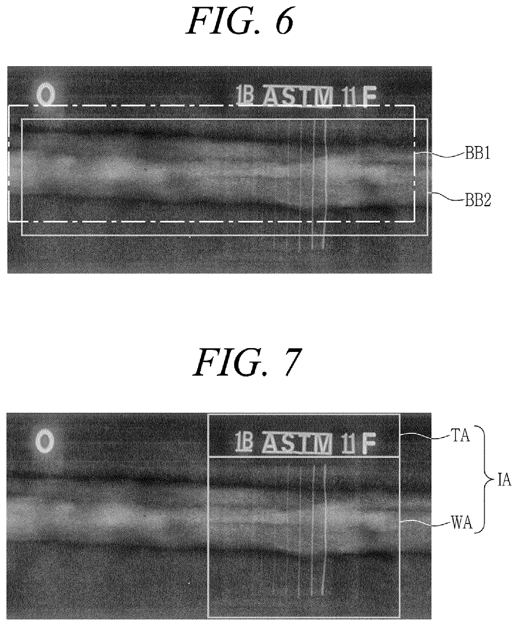

Next, a method for inspecting a defect in a weld on the basis of radiographic testing (RT) according to the exemplary embodiment of the present disclosure will be described. FIG. 5 is a flowchart illustrating the method for inspecting the defect in the weld on the basis of the radiographic testing according to the exemplary embodiment of the present disclosure. To emphasize, FIG. 5 is a detailed description of step S230 in FIG. 4. FIGS. 6 to 11 are screen examples illustrating the method for inspecting the defect in the weld on the basis of the radiographic testing according to the exemplary embodiment of the present disclosure.

Referring to FIG. 5, in step S310, the radiographic image may be input to the testing device 10. As described in FIG. 4, the radiographic image is obtained by converting the radiographic film derived through the radiography into a film in a digital format after attaching the Image Quality Indicator (IQI) to the target object on which the welding has been performed.

When the radiographic image is input in this way, the reading region processing unit 200 performs image processing so that the features of the reading region ROI including the welded part, i.e., a weld bead part, of the target object are highlighted in the radiographic image. Such image processing is to highlight a Region of Interest, i.e., the edges of the weld bead.

Next, in step S330, the reading region processing unit 200 detects the reading region ROI representing an area occupied by the weld bead in the radiographic image.

According to the exemplary embodiment of step S330, the reading region processing unit 200 may extract signals representing levels of pixels of the radiographic image through a detection model based on local extremum point search, detect edges representing the weld bead in the extracted signals from maximum and minimum points of the extracted signals, and detect an area including the edges representing the weld bead in the radiographic image as the reading region ROI through a bounding box BB1 as shown in FIG. 6.

According to another exemplary embodiment of step S330, as shown in FIG. 6, by using a reading region detection model that is a learning model, the reading region processing unit 200 may detect a reading region ROI, which is an area including all edges representing a weld bead in a radiographic image, through a bounding box BB2.

According to a yet another exemplary embodiment of step S330, a reading region ROI may be detected by using both a reading region detection model and a detection model based on local extremum point search. Specifically, first, the reading region processing unit 200 extracts signals representing levels of pixels of a radiographic image through the detection model based on the local extremum point search, detects edges representing a weld bead in the extracted signals from maximum and minimum points of the signals, and may detect an area including the edges representing the weld bead as the reading region ROI through a first bounding box BB1 in the radiographic image as shown in FIG. 6.

In addition, as shown in FIG. 6, by using the reading region detection model (RDM), the reading region processing unit 200 may detect an area including the edges representing the weld bead as the reading region ROI through a second bounding box BB2. Next, the reading region processing unit 200 may detect an intersection area of the first bounding box and the second bounding box as the final reading region ROI.

Next, in step S340, the image quality processing unit 300 performs image processing so that the features of the image quality indicator, i.e., in particular, the features of wires of the image quality indicator, are highlighted in the radiographic image. Specifically, when summarized, such image processing is described as follows.

The image quality processing unit 300 may perform a process of Contrast Limited Adaptive Histogram Equalization (CLAHE) on a wire area image so as to increase the sharpness of each edge in the radiographic image, thereby removing noise in the radiographic image and increasing brightness and contrast.

The image quality processing unit 300 may perform frequency domain filtering. In this case, the image quality processing unit 300 transforms a time domain radiographic image into an image in a frequency domain so as to generate a frequency domain image, performs frequency domain filtering on the frequency domain image by using a composite filter, and then reversely transforms the frequency domain image in the frequency domain into an image in the time domain. Here, the composite filter includes: a low-pass filter for allowing only low-band components of the frequency domain image to pass through; a vertical weight filter for multiplying a vertical component in the frequency domain image by a predetermined weight; a hourglass-shaped filter for eliminating upper and lower areas among areas divided by two different line segments passing through a reference point (e.g., a central point of a frequency domain image) while being vertically symmetrical with respect to the reference point in the frequency domain image; and a Butterworth filter for maximally reducing phase distortion in a boundary area of a frequency being filtered in the frequency domain image. In particular, the Butterworth filter is created according to the following

Equation:

$$G(w) = \frac{1}{\sqrt{1 + w^{2n}}}$$

Here, w denotes a frequency and n denotes a filtering order.

The image quality processing unit 300 performs filtering using the Sobel filter in both directions based on a vertical line segment with respect to a longitudinal direction of the edges in the radiographic image so that a boundary of a vertical component with respect to the longitudinal direction of the edges existing in the radiographic image is detected.

The image quality processing unit 300 binarizes a radiographic image, so as to derive a binarized radiographic image. In this case, the image quality processing unit 300 may binarize the radiographic image by using an upper pixel value having a predetermined ratio in a pixel-value distribution of the radiographic image as a threshold value. That is, the image quality processing unit 300 binarizes the radiographic image into pixels having pixel values greater than or equal to the threshold value and into the remaining pixels.

The image quality processing unit 300 performs a morphology operation on the radiographic image. In this case, the image quality processing unit 300 may perform at least one of an erosion operation for removing noise from the radiographic image and a dilatation operation for allowing each edge of the radiographic image to be expanded, or may perform the erosion operation and the dilatation operation sequentially.

Through such image processing, it may be possible for the image quality processing unit 300 to detect the same number of wires as the number of wires that are visually evaluated by an inspector.

Next, in step S350, the image quality processing unit 300 counts the number of wires of the image quality indicator in the radiographic image. Referring to FIG. 7, the image quality indicator includes: a text area TA composed of a text indicating a specification of the image quality indicator; and a wire area WA in which a plurality of wires having thicknesses different from each other is arranged so as to determine quality. An image quality indicator area IA is an area including the text area TA and the wire area WA.

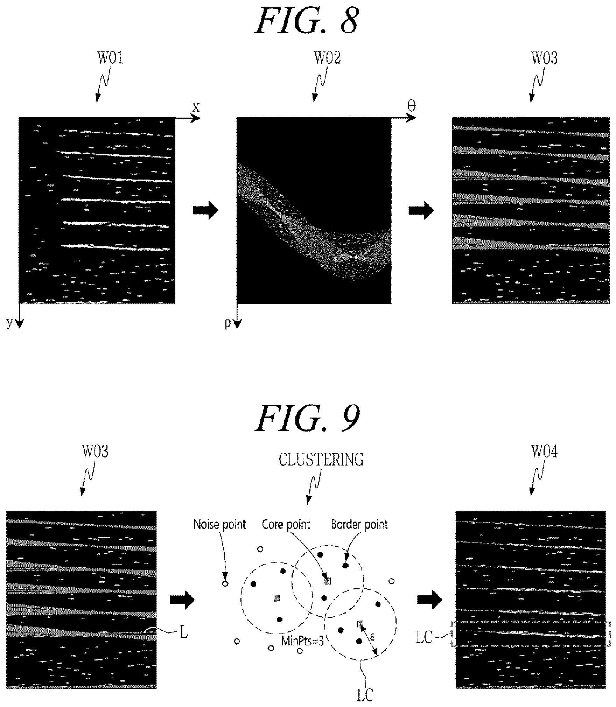

According to the exemplary embodiment of step S350, the image quality processing unit 300 detects the text in the radiographic image and specifies the text area TA, which is the area occupied by the text of the image quality indicator. In this case, Optical Character Recognition (OCR) may be used. In addition, the image quality processing unit 300 specifies the wire area WA, which is the area occupied by the plurality of wires of the image quality indicator, on the basis of the text area TA. Since the wire area WA is located in a predetermined area below the text area with respect to a direction of the text, the image quality processing unit 300 may specify the wire area WA with respect to the direction of the text in the specified text area. Next, the image quality processing unit 300 counts the number of wires of the image quality indicator in the wire area WA. To this end, the image quality processing unit 300 performs a Hough Transform on the wire area WA, so as to detect a plurality of lines in the wire area WA. To this end, as illustrated in FIG. 8, the image processing unit 300 converts an image WO1 of the wire area WA in a two-dimensional rectangular coordinate system (i.e., a Cartesian coordinate system) into an image in a polar coordinate system, so as to generate a polar coordinate system image WO2 having a plurality of curves. Then, the image quality processing unit 300 organizes points where the plurality of curves of the polar coordinate system image WO2 intersect into an accumulation array. Next, the image quality processing unit 300 creates an image WO3 of the wire area WA having the plurality of lines by mapping a plurality of points whose accumulation array values are greater than or equal to a threshold value, back to the image of the wire area WA in the rectangular coordinate system. In this way, as illustrated in FIG. 9, when the plurality of lines is detected, the image quality processing unit 300 clusters the plurality of lines on the basis of a density of the plurality of lines in the image WO3 of the wire area WA having the plurality of lines, so as to derive one or more line clusters LCs. Next, the image quality processing unit 300 counts the number of wires in the image quality indicator according to the number of line clusters LCs derived from an image WO4 of the wire area WA having the derived line clusters LCs.

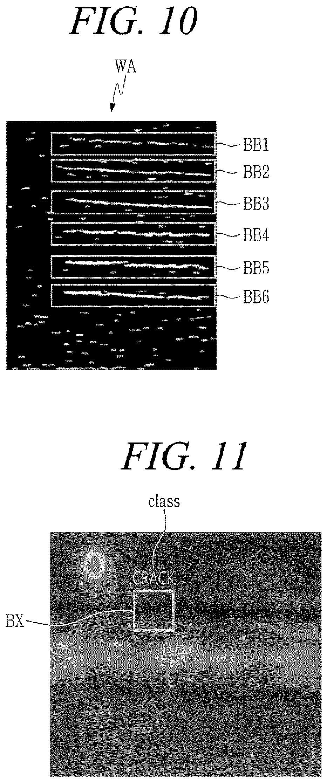

According to the exemplary embodiment of step S350, the image processing unit 300 detects the wire area WA, which is the area occupied by the plurality of wires of the image quality indicator in the radiographic image, through a bounding box by using the image quality indicator detection model. Then, as illustrated in FIG. 10, the image quality processing unit 300 detects a count wire area, which is an area occupied by each of the plurality of wires of the image quality indicator in the wire area WA, through bounding boxes BB1 to BB6 by using the count model, and counts the number of wires of the image quality indicator according to the number of detected count wire areas.

Next, in step S360, the image quality processing unit 300 evaluates whether the quality of the radiographic image satisfies a predefined quality condition according to the number of counted wires. In step S360, in a case where the number of wires counted previously in step S350 is greater than or equal to a reference value of the predefined quality condition, the image quality processing unit 300 may determine that the quality of the radiographic image is suitable for performing a defect test. In contrast, in a case where the number of wires counted in advance in step S350 is less than the reference value of the predefined quality condition, the quality processing unit 300 may determine that the quality of the radiographic image is unsuitable for performing the defect test. That is, as the quality of the radiographic image improves, the number of counted wires increases, and as the quality of the radiographic image decreases, the number of counted wires decreases. Accordingly, the quality of the radiographic image may be determined according to whether the number of counted wires is greater than or equal to the reference value of the predefined quality condition by comparing the number of counted wires with the predefined quality condition. In step S360, when it is determined that the quality of the radiographic image is suitable for performing the defect test, the quality processing unit 300 proceeds to step S380 so as to analyze the corresponding radiographic image and to start the defect test. In contrast, when it is determined that the quality of the radiographic image is unsuitable for performing the defect test, the image quality processing unit 300 outputs a rephotographing command for performing rephotographing of the target object in step S370.

When the quality of the radiographic image satisfies the preset reference value, that is, when the quality of the radiographic image is determined to be suitable for performing the defect test according to the number of counted wires, the defect processing unit 400 performs image processing to highlight the features of the defect in the welded region in the reading region ROI in step S380.

Then, in step S390, the defect processing unit 400 detects the defect in the welded part on the reading region ROI. In this case, the defect processing unit 400 derives a defect vector by performing weight calculations in which weights that have been learned are applied to the reading region ROI of the radiographic image. The defect vector includes the defect bounding box BB and the defect class. The defect bounding box represents the area occupied by the defect in the reading region ROI of the radiographic image. Such a defect bounding box BX may be expressed by the center coordinates, width, and height of the bounding box. The defect class represents the type of the detected defect. FIG. 11 shows an example of a case where the defect class is a crack class CRACK in which a crack occurs in a weld bead.

In step S400, when the defect is detected, the output unit 500 generates and outputs a defect report for indicating a defect location and a defect area through the defect bounding box and for indicating a defect type through the defect class. Such a defect report is provided to an inspector, who may refer to this report for further evaluation. Alternatively, additional evaluation may be performed for a defect obtained by matching and comparing the defect location, defect area, and defect class in the defect report with the defect location, defect area, and defect class, which are written by the inspector.

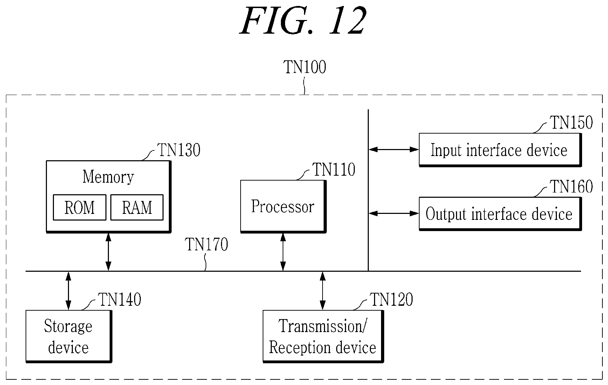

FIG. 12 is a view illustrating a computing device according to the exemplary embodiment of the present disclosure. The computing device TN100 in FIG. 12 may be a device (e.g., a testing device 10, etc.) described in the present specification.

In the exemplary embodiment of FIG. 12, the computing device TN100 may include at least one processor TN110, a transmission/reception device TN120, and a memory TN130. In addition, the computing device TN100 may further include a storage device TN140, an input interface device TN150, an output interface device TN160, etc. The components included in the computing device TN100 and connected to each other by a bus TN170 may communicate with each other.

The processor TN110 may execute program commands stored in at least one among the memory TN130 and the storage device TN140. The processor TN110 may refer to a central processing unit (CPU), a graphics processing unit (GPU), or a dedicated processor on which methods according to the exemplary embodiments of the present disclosure are performed. The processor TN110 may be configured to implement procedures, functions, methods, and the like which are described in conjunction with the exemplary embodiments of the present disclosure. The processor TN110 may control each component of the computing device TN100.

Each of the memory TN130 and the storage device TN140 may store various information related to the operation of the processor TN110. Each of the memory TN130 and the storage device TN140 may be comprised of at least one among a volatile storage medium and a non-volatile storage medium. For example, the memory TN130 may be composed of at least one of read only memory (ROM) and random access memory (RAM).

The transmission/reception device TN120 may transmit or receive wired signals or wireless signals. The transmission/reception device TN120 may be connected to a network and perform communication.

In particular, each of the above-described training unit 100, reading region processing unit 200, image quality processing unit 300, defect processing unit 400, and output unit 500 may be implemented in the form of a program readable by a computer means, be stored in the memory TN130, and then be executed in the processor TN110, or may be a sub-module of the processor TN110.

Meanwhile, various methods according to the exemplary embodiments of the present disclosure described above may be implemented in the form of programs readable through various computer means and be recorded on computer-readable recording media. Here, the recording media may store program commands, data files, data structures, etc., singly or in combination thereof. The program commands recorded on the recording media may be designed and configured specifically for the embodiments of the present disclosure or may be publicly known and available to those skilled in the art of computer software. For example, the recording media include: magnetic media such as hard disks, floppy disks, and magnetic tapes; optical media such as CD-ROMs and DVDs; magneto-optical media such as floptical disks; and a hardware device specially configured to store and execute program commands, the hardware device including such as ROM, RAM, flash memory, etc. Examples of the computer commands include not only machine language generated by a compiler, but also high-level language wires executable by a computer using an interpreter or the like. Such a hardware device described above may be configured to operate by using one or more software modules in order to perform the operation of the embodiments of the present disclosure, and vice versa.

Although the exemplary embodiments of the present disclosure have been described above, those skilled in the art will be able to modify and change the present disclosure in various ways by attaching, changing, deleting, or adding components without departing from the spirit of the present disclosure as described in the patent claims, and this will also be included within the scope of rights of the present disclosure.

**Claims**

**1.** A method for inspecting a defect in a weld, the method comprising:

    performing, by a reading region processing unit 200, image processing to highlight features of a reading region comprising a welded part of a target object in a radiographic image;
    detecting, by the reading region processing unit 200, the reading region in the radiographic image;

performing, by an image quality processing unit 300, image processing to highlight features of an image quality indicator in the radiographic image;

counting, by the image quality processing unit 300, a number of wires of the image quality indicator in the radiographic image;

evaluating, by the image quality processing unit 300, a quality of the radiographic image according to the number of wires of the image quality indicator;

performing, by a defect processing unit 400, image processing to highlight features of a defect in the welded part when the quality of the radiographic image satisfies a preset reference value;

detecting, by the defect processing unit 400, the defect in the welded part; and

outputting, by an output unit 500, a defect report comprising a defect location, a defect area, and a defect type according to the defect in the welded part.

2. The method of claim 1, wherein, in detecting the reading region, the reading region processing unit 200 extracts signals representing levels of pixels of the radiographic image through a local extremum point search-based detection model, detects edges representing a weld bead in the extracted signals from maximum and minimum points of the extracted signals, and detects an area occupied by the edges representing the weld bead in the radiographic image as the reading region through a bounding box.

3. The method of claim 1, wherein, in detecting of the reading region, the reading region processing unit 200 detects an area occupied by the reading region in the radiographic image by using a bounding box through a reading region detection model, wherein the reading region detection model is a learning model.

4. The method of claim 1, wherein detecting the reading region comprises:

extracting, by the reading region processing unit 200, signals that represent levels of pixels of the radiographic image through a local extremum point search-based detection model;

detecting, by the reading region processing unit 200, edges that represent a weld bead in the extracted signals from maximum and minimum points of the extracted signals;

detecting, by the reading region processing unit 200, an area occupied by the edges that represent the weld bead in the radiographic image as the reading region through a first bounding box;

detecting, by the reading region processing unit 200, an area occupied by the reading region through a second bounding box by using a reading region detection model; and

detecting, by the reading region processing unit 200, an intersection area of the first bounding box and the second bounding box as a final reading region.

5. The method of claim 1, wherein counting the number of wires of the image quality indicator comprises:

specifying, by the image quality processing unit 300, a text area, wherein the text area is an area occupied by a text of the image quality indicator in the radiographic image;

specifying, by the image quality processing unit 300, a wire area, wherein the wire area is an area occupied by the wires of the image quality indicator based on the text area; and

counting, by the image quality processing unit 300, the number of wires of the image quality indicator in the wire area.

6. The method of claim 5, wherein counting the number of wires of the image quality indicator in the wire area comprises:

performing, by the image quality processing unit 300, a Hough Transform on the wire area to create an image of the wire area having a plurality of lines;

clustering, by the image quality processing unit 300, the plurality of lines based on a density of the plurality of lines to derive one or more line clusters; and

counting, by the image quality processing unit 300, the number of wires of the image quality indicator according to a number of derived line clusters.

7. The method of claim 1, wherein counting the number of wires of the image quality indicator comprises:

detecting, by the image quality processing unit 300 using an image quality indicator detection model, a wire area that is an area occupied by a plurality of wires of the image quality indicator in the radiographic image through a bounding box; and

detecting, by the image quality processing unit 300 using a count model, each count wire area that is an area occupied by each of the plurality of wires of the image quality indicator in the wire area through a bounding box; and

counting, by the image quality processing unit 300 using the count model, the number of wires of the image quality indicator according to a number of detected count wire areas.

8. The method of claim 1, wherein, in detecting the defect in the welded part, the defect processing unit 400 derives a defect vector by performing weight calculations in which weights that have been learned are applied to the reading region of the radiographic image, and

the defect vector comprises a defect bounding box indicating an area occupied by the defect in the welded part and a defect class indicating a type of the defect in the welded part.

9. The method of claim 1, further comprising:

before the performing of the image processing to highlight the features of the reading region,

loading, by a training unit 100, training data comprising a training radiographic image and a target vector;

inputting, by the training unit 100, the training radiographic image into a defect detection model having weights that have not been learned;

performing, by the defect detection model, weight calculations in which the weights that have not been learned are applied to the training radiographic image to derive a defect vector comprising a defect bounding box for detecting an area occupied by a defect in the training radiographic image and a defect class for indicating a type of the defect in the training radiographic image;

calculating, by the training unit 100, a loss representing a difference between the target vector and the defect vector through a loss function; and

performing, by the training unit 100, optimization for modifying the weights of the defect detection model to reduce the calculated loss.

10. The method of claim 9, wherein the training radiographic image comprises a radiographic image of a target object having a defect in a welded part that is generated by synthesizing an image of a defect part with a radiographic image of the target object having no defect in the welded part, and

the target vector comprises a target bounding box for indicating the area occupied by the defect in the training radiographic image and a target class for indicating a type of the defect in the training radiographic image.

11. A device for inspecting a defect in a weld, the device comprising:

a reading region processing unit 200 configured to perform image processing to highlight features of a reading region that comprises a welded part of a target object in a radiographic image and detect the reading region in the radiographic image;

an image quality processing unit 300 configured to perform image processing to highlight features of an image quality indicator in the radiographic image, count a number of wires of the image quality indicator in the radiographic image, and evaluate a quality of the radiographic image according to the number of wires of the image quality indicator;

a defect processing unit 400 configured to perform image processing to highlight features of a defect in the welded part when the quality of the radiographic image satisfies a preset reference value and detect the defect in the welded part; and

an output unit 500 configured to output a defect report that comprises a defect location, a defect area, and a defect type according to the defect in the welded part.

12. The device of claim 11, wherein the reading region processing unit 200 is configured to:

extract signals representing levels of pixels of the radiographic image through a local extremum point search-based detection model;

detect edges representing a weld bead in the extracted signals from maximum and minimum points of the extracted signals; and

detect an area occupied by the edges representing the weld bead in the radiographic image as the reading region through a bounding box.

13. The device of claim 11, wherein the reading region processing unit 200 is configured to detect an area occupied by the reading region in the radiographic image by using a bounding box through a reading region detection model, wherein the reading region detection model is a learning model.

14. The device of claim 11, wherein the reading region processing unit 200 is configured to:

extract signals representing levels of pixels of the radiographic image through a local extremum point search-based detection model;

detect edges representing a weld bead in the extracted signals from maximum and minimum points of the extracted signals;

detect an area occupied by the edges representing the weld bead in the radiographic image as the reading region through a first bounding box;

detect an area occupied by the reading region through a second bounding box by using a reading region detection model; and

detect an intersection area of the first bounding box and the second bounding box as a final reading region.

15. The device of claim 11, wherein the image quality processing unit 300 is configured to:

specify a text area, wherein the text area is an area occupied by a text of the image quality indicator in the radiographic image;

specify a wire area, wherein the wire area is an area occupied by the wires of the image quality indicator based on the text area; and

count the number of wires of the image quality indicator in the wire area.

# FIG. 1

```
┌─────────────────┐        ┌─────────────┐        ┌─────────────────┐
│  ⌐RTA           │        │  ⌐SC        │        │  ⌐10            │
│  Radiographic   │───────▶│   Scanner   │───────▶│  Testing device │
│  device         │        │             │        │                 │
└─────────────────┘        └─────────────┘        └─────────────────┘
```

# FIG. 2

10

```
┌─────────────────┐
│  ⌐100           │
│  Training unit  │
└────────┬────────┘
         │
    ┌────┼──────────────────┐
    ▼                ▼        ▼
┌──────────┐  ┌──────────┐  ┌──────────┐        ┌──────────┐
│ ⌐200     │  │ ⌐300     │  │ ⌐400     │        │ ⌐500     │
│ Reading  │  │ Image    │  │ Defect   │        │ Output   │
│ region   │─▶│ quality  │─▶│ processing│──────▶│ unit     │
│ processing│  │ processing│  │ unit     │        │          │
│ unit     │  │ unit     │  │          │        │          │
└──────────┘  └──────────┘  └──────────┘        └──────────┘
```

# FIG. 3

```
                                              ┌─ S110
┌──────────────────────────────────────────┐
│            Creating training data          │
└──────────────────────────────────────────┘
                    │
                    ▼                         ┌─ S120
┌──────────────────────────────────────────┐
│       Inputting training radiographic image │◄─────┐
└──────────────────────────────────────────┘       │
                    │                               │
                    ▼                         ┌─ S130 │
┌──────────────────────────────────────────┐       │
│            Detecting defect vector         │       │
└──────────────────────────────────────────┘       │
                    │                               │
                    ▼                         ┌─ S140 │
┌──────────────────────────────────────────┐       │
│   Deriving loss representing difference    │       │
│  between target vector and defect vector   │       │
└──────────────────────────────────────────┘       │
                    │                               │
                    ▼                         ┌─ S150 │
┌──────────────────────────────────────────┐       │
│             Modifying weights              │       │
└──────────────────────────────────────────┘       │
                    │                               │
                    ▼                         ┌─ S160 │
              ◇ Is condition satisfied? ◇────────────┘
                    │                          No
                Yes │
                    ▼                         ┌─ S170
┌──────────────────────────────────────────┐
│             Completing training            │
└──────────────────────────────────────────┘
```

# FIG. 4

$\overset{\text{S210}}{\diagup}$

| Deriving radiographic testing film through radiography |
|---|

$\overset{\text{S220}}{\diagup}$

| Digitizing radiographic image |
|---|

$\overset{\text{S230}}{\diagup}$

| Performing inspection by using digitized radiographic image |
|---|

## FIG. 5

```
                                            ┌─ S310
┌──────────────────────────────────────┐
│  Inputting digitized radiographic image │
└──────────────────────────────────────┘
                    │
                    ▼                       ┌─ S320
┌──────────────────────────────────────┐
│  Performing image processing so as to   │
│       highlight reading region          │
└──────────────────────────────────────┘
                    │
                    ▼                       ┌─ S330
┌──────────────────────────────────────┐
│        Detecting reading region         │
└──────────────────────────────────────┘
                    │
                    ▼                       ┌─ S340
┌──────────────────────────────────────┐
│  Performing image processing so as to   │
│ highlight features of image quality indicator │
└──────────────────────────────────────┘
                    │
                    ▼                       ┌─ S350
┌──────────────────────────────────────┐
│    Counting number of wires in image    │
│         quality indicator               │
└──────────────────────────────────────┘
                    │
                    ▼                       ┌─ S360
           ◇ Is quality condition satisfied? ◇ ────── No ──┐
                    │                                        │
                   Yes                                       │
                    ▼                       ┌─ S380          │
┌──────────────────────────────────────┐                    │
│  Performing image processing so as to   │                  │
│       highlight features of defect      │                  │
└──────────────────────────────────────┘                    │
                    │                                        │
                    ▼                       ┌─ S390          │
┌──────────────────────────────────────┐                    │
│            Detecting defect             │                  │
└──────────────────────────────────────┘                    ▼
                    │            ┌─ S400                         ┌─ S370
                    ▼                              ┌──────────────────────────────────┐
┌──────────────────────────────────────┐          │  Outputting rephotographing command │
│      Outputting defect information      │          └──────────────────────────────────┘
└──────────────────────────────────────┘
```

## FIG. 6

## FIG. 7

# FIG. 8

W01

W02

W03

# FIG. 9

W03

CLUSTERING

W04

Noise point    Core point    Border point

MinPts=3

L

LC

LC

# FIG. 10

WA

BB1
BB2
BB3
BB4
BB5
BB6

# FIG. 11

class

CRACK

BX

## FIG. 12

TN100

Memory (TN130)
- ROM
- RAM

Processor (TN110)

Input interface device (TN150)

Output interface device (TN160)

TN170

Storage device (TN140)

Transmission/Reception device (TN120)

| | Europäisches Patentamt European Patent Office Office européen des brevets | **EUROPEAN SEARCH REPORT** | **Application Number** EP 25 17 2332 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 115 266 774 A (CHINA SPECIAL EQUIPMENT INSPECTION & RES INST) 1 November 2022 (2022-11-01) | 1,5-11, 15 | INV. G06T7/11 G06T7/187 |
| Y | * page 1 - page 3 * | 2-4, 12-14 | G06T7/00 G06T7/12 |
| | ----- | | |
| X | KR 2024 0030441 A (KOREA INST ROBOT & CONVERGENCE [KR]) 7 March 2024 (2024-03-07) | 1,3, 5-11,13, 15 | |
| Y | * paragraphs [0033] - [0041], [0049] - [0051], [0056] - [0065]; figure 5 * | 2,4,12, 14 | |
| | ----- | | |
| Y | YANG WEI ET AL: "Generalized weld bead region of interest localization and improved faster R-CNN for weld defect recognition", MEASUREMENT., vol. 222, 1 October 2023 (2023-10-01), page 113619, XP093316681, GB ISSN: 0263-2241, DOI: 10.1016/j.measurement.2023.113619 Retrieved from the Internet: URL:https://pdf.sciencedirectassets.com/27 1454/1-s2.0-S0263224123X00165/1-s2.0-S0263 224123011831/main.pdf?hash=c71b1f739e875d6 c38db47de4ad716d1ef3ffb241a46e2b807ecb83d1 e977afa&host=68042c943591013ac2b2430a89b27 0f6af2c76d8dfd086a07176afe7c76c2c61&pii=S0 263224123011831&tid=spdf-62ca7304-b075-4a6 c-93e2-a5d> [retrieved on 2025-09-19] * Sections 2.2, 3.1 * | 2-4, 12-14 | TECHNICAL FIELDS SEARCHED (IPC) G06T |
| | ----- -/-- | | |

The present search report has been drawn up for all claims

| Place of search | Date of completion of the search | Examiner |
|---|---|---|
| Munich | 21 September 2025 | Tibrewal-Fabricius |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

...............................................................................
& : member of the same patent family, corresponding document

EPO FORM 1503 03.82 (P04C01)

**page 1 of 2**

Europäisches
Patentamt

European
Patent Office

Office européen
des brevets

**EUROPEAN SEARCH REPORT**

**Application Number**

EP 25 17 2332

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | PIOTR BANIUKIEWICZ ET AL: "Automatic Detection and Identification of Image Quality Indicators in Radiograms", STUDIES IN APPLIED ELECTROMAGNETICS AND MECHANICS / ELECTROMAGNETIC NONDESTRUCTIVE EVALUATION (XIV), AMSTERDAM [U.A.] : IOS PRESS, 2011, NL, PAGE(S) 157 - 163 , 1 January 2011 (2011-01-01), XP009501924, ISBN: 978-1-60750-749-9 Retrieved from the Internet: URL:http://ebooks.iospress.nl/volumearticl e/22938 [retrieved on 2025-09-19] * page 6 * ----- | 5-7,15 | |
| A | CN 109 859 177 B (AEROSPACE XINCHANGZHENG DADAO TECH CO LTD) 10 March 2023 (2023-03-10) * page 6 - page 7 * ----- | 1-15 | |
| | | | TECHNICAL FIELDS SEARCHED (IPC) |

The present search report has been drawn up for all claims

| Place of search | Date of completion of the search | Examiner |
|---|---|---|
| Munich | 21 September 2025 | Tibrewal-Fabricius |

EPO FORM 1503 03.82 (P04C01)

## ANNEX TO THE EUROPEAN SEARCH REPORT
## ON EUROPEAN PATENT APPLICATION NO.

EP 25 17 2332

21-09-2025

| Patent document cited in search report | | Publication date | Patent family member(s) | Publication date |
|---|---|---|---|---|
| CN 115266774 | A | 01-11-2022 | NONE | |
| KR 20240030441 | A | 07-03-2024 | NONE | |
| CN 109859177 | B | 10-03-2023 | NONE | |

EPO FORM P0459